# EUROPÄISCHE PATENTSCHRIFT

(11) **EP 3 752 053 B1**
(45) Veröffentlichungstag und Bekanntmachung des Hinweises auf die Patenterteilung: **15.01.2025**
(21) Anmeldenummer: 19705514.8
(22) Anmeldetag: 15.02.2019
(51) Int. Cl.: A61B 5/021, A61B 5/00, G06K 19/07, G06K 7/10

(54) **TRANSPONDERSYSTEM UND VERFAHREN ZUM AUSLESEN EINES PASSIVEN TRANSPONDERS**
TRANSPONDER SYSTEM AND METHOD FOR READOUT OF A PASSIVE TRANSPONDER
SYSTÈME DE TRANSPONDEUR ET PROCÉDÉ DE LECTURE D'UN TRANSPONDEUR PASSIF

(30) Priorität: 16.02.2018 DE 102018202430
(43) Veröffentlichungstag der Anmeldung: 23.12.2020
(73) Patentinhaber: VesselSens GmbH, 53175 Bonn (DE)
(72) Erfinder: DOMNICH, Alexej, 53639 Königswinter (DE); TUTSCH, Fabian, 53175 Bonn (DE)
(74) Vertreter: Pfenning, Meinig & Partner mbB
(86) Internationale Anmeldenummer: PCT/EP2019/053840
(87) Internationale Veröffentlichungsnummer: WO 2019/158703

(56) Entgegenhaltungen:
- EP-A1- 1 854 406
- EP-A2- 1 990 027
- WO-A2-2006/049796
- DE-A1- 102011 009 695
- DE-A1- 19 733 360

## Beschreibung

Die Erfindung betrifft ein Transpondersystem mit zumindest einem passiven Transponder, der einen Schwingkreis mit veränderlicher Resonanzfrequenz aufweist, sowie mit einer Auslesevorrichtung, wobei die Auslesevorrichtung eingerichtet ist, eine Frequenz des Auslesesignals an die veränderliche Resonanzfrequenz des Schwingkreises anzupassen. Die Erfindung betrifft außerdem ein Verfahren zum Auslesen eines passiven Transponders, der einen Schwingkreis mit veränderlicher Resonanzfrequenz aufweist.

Passive Transponder enthalten normalerweise einen Schwingkreis, der dadurch anregbar ist, dass mittels einer Auslesevorrichtung ein elektromagnetisches Wechselfeld erzeugt wird, dessen Frequenz die Resonanzfrequenz des Transponders ist.

Derartige passive Transponder können unter anderem für Überwachungsaufgaben eingesetzt werden. In diesem Fall kann die Resonanzfrequenz des Schwingkreises von einem zu überwachenden Umgebungszustand abhängig gemacht werden. Eine solche Abhängigkeit kann beispielsweise dadurch hergestellt werden, dass Sensoren eingesetzt werden, die eine Induktivität oder eine Kapazität abhängig von einer zu überwachenden Größe verändern. Wird ein solcher Sensor in dem Schwingkreis des passiven Transponders eingesetzt, verändert sich dessen Resonanzfrequenz abhängig vom Wert der zu überwachenden Größe. Wird dann der passive Transponder von außen mit einem anregenden elektromagnetischen Wechselfeld beaufschlagt, so hängt das Auftreten einer Resonanz davon ab, ob die vom Wert des Umgebungszustands abhängige Resonanzfrequenz gleich der Resonanzfrequenz der Anregung ist. Wird der Resonanzzustand als Sollzustand vorgesehen, so ist eine Abweichung vom Sollzustand dadurch zu erkennen, dass der Schwingkreis des Transponders nicht mehr resoniert. Auf diese Weise kann das Bestehen des Sollzustandes überwacht werden.

Derartige Systeme erlauben eine Entscheidung, ob der durch den Sensor gemessene Wert ein Sollwert ist oder nicht. Befindet sich das System in Resonanz, so kann geschlossen werden, dass der Wert den Sollwert hat. Befindet sich das System außer Resonanz, so kann geschlossen werden, dass der Wert vom Sollwert abweicht. Die Systeme des Standes der Technik erlauben jedoch keine kontinuierliche Messung des Wertes. Nur in dem Fall, wo der Wert den Sollwert hat, ist der Wert bekannt. Weicht der vom Sensor zu messende Wert vom Sollwert ab, so ist er im Stand der Technik unbekannt.

Die WO 2006/049796 A2 beschreibt ein Verfahren zur Kommunikation mit einem implantierten drahtlosen Sensor. Dabei ist in dem Sensor ein kapazitiver Sensor mit einer Induktivität zu einem Schwingkreis verschaltet.

Die DE 10 2011 009 695 A1 beschreibt eine zylinderförmige Vorrichtung zum Durchfluss von Fluid, die zumindest zwei Drucksensoren aufweist, mit welchen ein Druck des Fluids im Inneren der Vorrichtung messbar ist. Die Drucksensoren sind dabei kapazitive Drucksensoren.

Die EP 1 990 027 A2 beschreibt ein medizinisches Implantat, insbesondere einen Stent, zum Einsatz im Körperlumen. Das Implantat weist eine passive elektronische Schwingkreisanordnung mit einer Induktivität und einem Kondensator auf, deren Eigenfrequenz im implantierten Zustand von außen durch Anregung des Schwingkreises erfassbar ist.

DE 197 33 360 A1 beschreibt einen Feuchtigkeitssensor zum Nachweis von Regenwasser auf einer Windschutzscheibe eines Kraftfahrzeuges. Der Feuchtigkeitssensor besteht im Wesentlichen aus einer Spulenanordnung, die eine auf einem Isolierstoffträger flach aufliegende, spiralförmig gewickelte Spule umfasst.

EP 1 854 406 A1 D5 beschreibt ein System zur Positionsbestimmung. Das System umfasst einen Feldgenerator, einen Transponder und einen Positionsverfolger. Der Feldgenerator erzeugt ein positionsveränderliches Magnetfeld. Der Transponder umfasst einen Schwingkreis mit einer Resonanzfrequenz und einer Induktionsspule, die die Resonanzfrequenz abhängig vom positionsver-änderlichen Magnetfeld verändert. Der Positionsverfolger erfasst die Resonanzfrequenz und bestimmt eine Position des Transponders abhängig von der erfassten Resonanzfrequenz.

Aufgabe der vorliegenden Erfindung ist es, ein Transpondersystem anzugeben, mit dem in einem passiven Transponder, dessen Schwingkreis eine veränderliche Resonanzfrequenz aufweist, die veränderliche Resonanzfrequenz bestimmbar ist. Aufgabe ist es außerdem, ein Verfahren zum Auslesen eines passiven Transponders anzugeben, das es bei einem passiven Transponder mit veränderlicher Resonanzfrequenz erlaubt, diese Resonanzfrequenz zu bestimmen.

Die Aufgabe wird gelöst durch das Transpondersystem nach Anspruch 1 und das Verfahren zum Auslesen eines Transponders nach Anspruch 7. Die jeweiligen abhängigen Ansprüche geben vorteilhafte Weiterbildungen des erfindungsgemäßen Transpondersystems und des erfindungsgemäßen Verfahrens zum Auslesen eines passiven Transponders an.

Erfindungsgemäß wird ein Transpondersystem angegeben, das zumindest einen passiven Transponder aufweist. Der passive Transponder enthält dabei zumindest einen Schwingkreis, der eine veränderliche Resonanzfrequenz hat. Dass die Resonanzfrequenz veränderlich ist, kann dabei so verstanden werden, dass der Wert der Resonanzfrequenz sich bei bestimmungsgemäßen Einsatz des Transponders mit der Zeit verändert, also zeitlich veränderlich ist. Besonders bevorzugt ist die Resonanzfrequenz stetig veränderlich mit der Zeit.

Das erfindungsgemäße Transpondersystem weist außerdem zumindest eine Auslesevorrichtung auf, die zumindest eine Ausleseinduktivität hat. Mit der Ausleseinduktivität ist der zumindest eine Schwingkreis des passiven Transponders mit einem Auslesesignal beaufschlagbar. Das Auslesesignal kann dabei ein elektromagnetisches Wechselfeld sein. Erfindungsgemäß ist die Auslesevorrichtung eingerichtet, eine Frequenz des Auslesesignals an die zeitlich veränderliche Resonanzfrequenz des Schwingkreises anzupassen. Dadurch kann die Auslesevorrichtung den Schwingkreis stets mit einem Auslesesignal beaufschlagen, dessen Frequenz gleich der Resonanzfrequenz des Schwingkreises zum Zeitpunkt der Beaufschlagung ist. Verändert sich die Resonanzfrequenz des Schwingkreises, kann die Auslesevorrichtung die Frequenz des Auslesesignals an die veränderte Resonanzfrequenz anpassen. Dadurch, dass die Frequenz des Auslesesignals an die Resonanzfrequenz des Schwingkreises angepasst wird, ist die Resonanzfrequenz des Schwingkreises mit der Ausleseeinheit bestimmbar. Auf diese Weise kann also bei einer veränderlichen Resonanzfrequenz des Schwingkreises des Transponders der Wert der Resonanzfrequenz bestimmt werden. Vorteilhaft ist es dabei, wenn die Frequenz des Auslesesignals der veränderlichen Resonanzfrequenz des Schwingkreises stetig nachgeführt wird, da hierdurch eine kontinuierliche Kenntnis der Resonanzfrequenz ermöglicht wird.

In einer vorteilhaften Ausgestaltung der Erfindung kann die Resonanzfrequenz des Schwingkreises dadurch veränderlich sein, dass eine Induktivität oder eine Kapazität des Schwingkreises durch einen Messwert eines Sensors einstellbar ist. Verändert sich der Sensorwert, so verändert sich die Kapazität bzw. die Induktivität und dadurch die Resonanzfrequenz des Schwingkreises. Vorteilhafterweise kann daher zumindest eine Induktivität zusammen mit zumindest einem kapazitiven Sensor oder zumindest ein induktiver Sensor mit zumindest einer Kapazität zu dem zumindest einen Schwingkreis des Transponders verschaltet sein. Besonders vorteilhaft ist eine Ausgestaltung, in der ein kapazitiver Drucksensor mit einer Induktivität zu dem Schwingkreis verschaltet ist. Kapazitive Drucksensoren können zumindest eine Kapazität bzw. einen Kondensator aufweisen. Die Kapazität kann sich dann in Abhängigkeit vom Druck, mit welchem der Drucksensor beaufschlagt wird, ändern. Ein einfaches Beispiel eines solchen kapazitiven Drucksensors ist ein Kondensator mit zwei elektrisch leitenden Flächen, deren Abstand vom Umgebungsdruck abhängig ist. Hierzu können die beiden Flächen beispielsweise auf gegenüberliegenden Flächen eines druckdicht abgeschlossenen Gehäuses angeordnet sein.

Die Ausgestaltung mit zumindest einem kapazitiven Drucksensor ist besonders vorteilhaft, wenn der passive Transponder zur Druckmessung von Fluid, beispielsweise in einem Fluidleiter, eingesetzt wird. Das erfindungsgemäße Transpondersystem erlaubt es dann, den Druck in der Fluidleitung zu messen, ohne zur Messung in die Fluidleitung eingreifen zu müssen. Eine solche Fluidleitung kann beispielsweise ein Blutgefäß sein. In diesem Fall ist es besonders vorteilhaft, dass der Druck ohne einen Eingriff in das Blutgefäß gemessen werden kann.

In einer vorteilhaften Ausgestaltung der Erfindung kann die Auslesevorrichtung eine Anregungseinheit zur Erzeugung eines Auslesesignals und eine Rückmeldeeinheit zur Erfassung eines vom passiven Transponder ausgehenden Rückmeldesignals aufweisen. Die Rückmeldeeinheit kann dann eingerichtet sein, ein Frequenzanpassungssignal an die Anregungseinheit zu übergeben, das basierend auf dem Rückmeldesignal ermittelt wird. Die Anregungseinheit kann dann die Frequenz des Auslesesignals basierend auf dem Frequenzanpassungssignal anpassen. Es kann hier also ein Auslesesignal erzeugt werden, mit dem der passive Transponder beaufschlagt wird. Es geht dadurch vom passiven Transponder ein Rückmeldesignal aus, das von der Auslesevorrichtung aufgenommen wird. Ergibt sich aus dem Rückmeldesignal, dass sich die Resonanzfrequenz des Schwingkreises verändert hat, so kann die Frequenz des Auslesesignals entsprechend angepasst werden. Auf diese Weise kann die Frequenz des Auslesesignals an die Resonanzfrequenz des Schwingkreises angepasst werden bzw. diesem nachgeführt werden.

Die Anregungseinheit kann hierzu vorzugsweise eine Signalquelle aufweisen, mit der das Auslesesignal erzeugbar ist. Das Auslesesignal kann dabei mit einer bestimmten Frequenz erzeugt werden. Vorteilhafterweise kann die Ausleseeinheit außerdem einen Richtkoppler aufweisen, an dessen Ausgang die Signalquelle elektrisch gekoppelt ist. Das Signal mit der gegebenen Frequenz wird also an den Ausgang der Signalquelle gegeben. Am Eingang des Richtkopplers kann vorteilhafterweise die Ausleseinduktivität elektrisch angekoppelt sein. Auf diese Weise kann das von der Signalquelle erzeugte Signal mit der gegebenen Frequenz an die Ausleseinduktivität übertragen werden und/oder das von der Ausleseeinheit reflektierte Signal zurück an den Eingang der Rückmeldeeinheit übertragen werden.

In einer vorteilhaften Ausgestaltung der Erfindung kann die Rückmeldeeinheit einen Mischer aufweisen, mit dem ein durch die Ausleseinduktivität vom passiven Transponder empfangenes Signal mit dem Auslesesignal mischbar oder multiplizierbar ist. Auf diese Weise kann das durch die Ausleseinduktivität vom passiven Transponder empfangene Signal auf die Anregungsfrequenz gefiltert werden, so dass Abweichungen der Frequenz des vom passiven Transponder empfangenen Signals von dem Auslesesignal ermittelbar sind. Vorteilhafterweise kann die Rückmeldeeinheit außerdem einen Tiefpassfilter aufweisen, mit dem ein vom Mischer ausgegebenes Signal filterbar ist. Hierdurch können hochfrequentes Rauschen und andere Störsignale herausgefiltert werden. Insbesondere kann vorteilhafterweise die Rückmeldeeinheit als Lock-in-Verstärker ausgestaltet sein. Der aktuelle Wert der Resonanzfrequenz kann hierbei dem Regelkreis rückgeführt werden und die Frequenz des Auslesesignals kann so eingestellt werden, dass die Abweichung der Frequenz des Auslesesignals verringert oder aufgehoben wird.

Das erfindungsgemäße Transpondersystem ist besonders vorteilhaft einsetzbar zur Messung von Druck in Fluidleitungen. Durch die Messung von Druck in einer Fluidleitung kann insbesondere eine Geschwindigkeit von Pulswellen im Fluid innerhalb der Fluidleitung bestimmt werden. Hierzu kann der passive Transponder vorteilhafterweise zumindest zwei Schwingkreise aufweisen, die wie beschrieben ausgestaltet sein können. Insbesondere können die zumindest zwei Schwingkreise jeweils als Kapazität zumindest einen kapazitiven Drucksensor aufweisen. Der passive Transponder kann also einen ersten Schwingkreis mit einem ersten kapazitiven Drucksensor als Kapazität aufweisen und darüberhinaus einen zweiten Schwingkreis mit einem zweiten kapazitiven Drucksensor als Kapazität. In dieser Ausgestaltung kann dann das Transpondersystem eine zylinderförmige Vorrichtung zum Durchfluss von Fluid in Richtung einer Zylinderachse der zylinderförmigen Vorrichtung aufweisen. Die Zylinderform kann in den meisten Fällen eine Kreiszylinderform sein, sie kann jedoch auch jede andere Zylinderform umfassen. Die Fluidleitung kann insbesondere auch eine Flüssigkeitsleitung oder eine Gasleitung sein.

Unter einem Fluid kann hier eine Substanz verstanden werden, die einer beliebig langsamen Scherung keinen Widerstand entgegensetzt, die also eine endliche Viskosität hat. Insbesondere sind Gase und Flüssigkeiten Fluide in diesem Sinne.

Die kapazitiven Drucksensoren der zumindest zwei Schwingkreise können dann an unterschiedlichen Stellen der zylinderförmigen Vorrichtung entlang der Zylinderachse angeordnet sein. Es kann also der kapazitive Drucksensor des ersten Schwingkreises an einer ersten Stelle der zylinderförmigen Vorrichtung angeordnet sein und die Kapazität des zweiten Schwingkreises an einer von dieser entlang der Zylinderachse beabstandeten zweiten Stelle der zylinderförmigen Vorrichtung angeordnet sein. Auf diese Weise ist mit den zumindest zwei kapazitiven Drucksensoren ein Druck des Fluids an unterschiedlichen Stellen der zylinderförmigen Vorrichtung entlang der Zylinderachse messbar. Die zylinderförmige Vorrichtung kann beispielsweise ein Stent, ein Implantat, ein Katheter und/oder eine Fluidleitung sein, die auch flexibel sein kann.

Eine derartige Ausgestaltung des Transponders ermöglicht eine Messung des Druckes in der zylinderförmigen Vorrichtung an unterschiedlichen Stellen. Hierdurch kann insbesondere eine Geschwindigkeit von Pulswellen bestimmt werden, die die zylinderförmige Vorrichtung durchlaufen. Eine Pulswelle führt an einem gegebenen Ort entlang der Zylinderachse zu einer zeitlichen Veränderung des Druckes. Wird der Druck an unterschiedlichen Stellen gemessen, so führt die Druckwelle an den unterschiedlichen Orten nacheinander zu einer Veränderung des Druckes. Eine Zeitdifferenz der Druckveränderung kann mit den beiden Schwingkreisen bestimmt werden und aus einem bekannten Abstand der Drucksensoren kann dann die Druckwellengeschwindigkeit berechnet werden.

Es hat sich als vorteilhaft erwiesen, wenn in dem Fall, dass der passive Transponder mehrere Schwingkreise aufweist, diese voneinander verschiedene Resonanzfrequenzen haben. Hierdurch können die Signale der beiden Schwingkreise beim Auslesen voneinander unterschieden werden. Besonders vorteilhaft ist es dabei, wenn die Resonanzfrequenzen sich zumindest um einen solchen Betrag unterscheiden, dass sie sich nicht schwebend überlagern. Hängen die Resonanzfrequenzen wie oben beschrieben von Messwerten eines oder mehrerer Sensoren ab, so ist die vorteilhafte Bedingung, dass sich die Resonanzfrequenzen nicht schwebend überlagern, vorzugsweise für alle zu erwartenden oder auftretenden Messwerte und damit verbundenen Resonanzfrequenzen erfüllt. Weisen die Schwingkreise also beispielsweise kapazitive Drucksensoren wie vorstehend beschrieben auf, so sind die Schwingkreise vorteilhafterweise so eingerichtet, dass ihre Resonanzfrequenzen sich für alle zu messenden Drücke nicht schwebend überlagern.

Die Drucksensoren können vorteilhaft im gleichen Abstand von der Zylinderachse angeordnet sein. Es ist möglich aber nicht wesentlich, dass die Drucksensoren in der gleichen Richtung von der Zylinderachse aus gesehen angeordnet sind.

Erfindungsgemäß ist außerdem ein Verfahren zum Auslesen einer passiven Transponders, wobei der passive Transponder zumindest einen Schwingkreis mit einer veränderlichen Resonanzfrequenz aufweist. Es wird dabei der zumindest eine Schwingkreis mit einem Auslesesignal beaufschlagt und eine Frequenz des Auslesesignals an die veränderliche Resonanzfrequenz des Schwingkreises angepasst. Die vorstehend zum passiven Transpondersystem gemachten Aussagen gelten für das Verfahren entsprechend.

Vorteilhafterweise wird die Frequenz des Auslesesignals der veränderlichen Resonanzfrequenz des Schwingkreises stetig nachgeführt. Die Frequenz macht also vorzugsweise keine Sprünge. Bevorzugterweise wird die Frequenz des Auslesesignals der Resonanzfrequenz des passiven Transponders mittels eines Regelkreises so nachgeführt, dass die Frequenz des Auslesesignals gerade die jeweils gegenwärtige Resonanzfrequenz des Schwingkreises ist.

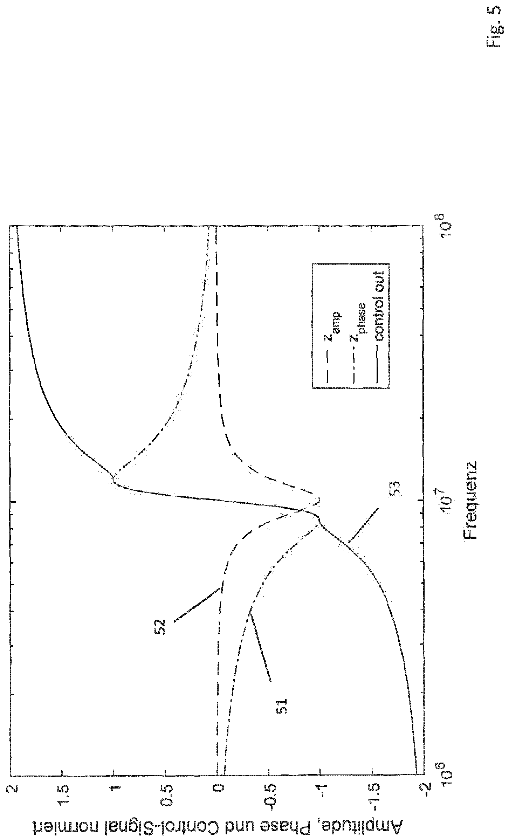

Vorteilhafterweise kann das Verfahren mittels eines Lock-In-Verstärkers ausgeführt werden. Es kann hierbei ein vom passiven Transponder ausgehendes Rückmeldesignal mit der Ausleseeinheit empfangen werden. Das empfangene Rückmeldesignal kann dann einem Eingang eines Lock-In-Verstärkers zugeführt werden. Ein Referenzsignal des Lock-In-Verstärkers kann dabei das Auslesessignal sein. Als Ausgang des Lock-In-Verstärkers ergeben sich Phase und Amplitude des Rückmeldesignals. Diese können dem Regelkreis zugeführt werden, so dass die Frequenz des Auslesesignals basierend auf der Magnitude bzw. Amplitude und der Phase des Rückmeldesignals korrigiert werden kann. Ist die Frequenzänderung im Vergleich zu einer Bandbreite der Sensorresonanz klein, so kann das Phasensignal als Eingangsgröße für einen Sollwert-Istwert-Vergleich im Regelkreis verwendet werden. Optional kann aus dem Phasen- und dem Amplitudensignal, also dem gesamten komplexen Signal, eine Größe gebildet werden, mittels derer die Regelung erfolgt. Auf diese Weise kann auch bestimmt werden, ob die Abweichung des Rückmeldesignals vom Auslesesignal positiv oder negativ ist. Auch die Verwendung der Phase ist möglich, wobei hier vorteilhafterweise aus der Phase und deren Ableitung eine neue Funktion zusammengesetzt werden kann, die die Beurteilung erlaubt wie stark die Frequenz positiv oder negativ abweicht (Siehe Fig. 5). Auch die Verwendung der Amplitude ist möglich, wobei hier dann vorzugsweise auch die Ableitung der Amplitude nach der Frequenz betrachtet wird, um entscheiden zu können, ob die Abweichung des Rückmeldesignals vom Auslesesignal positiv oder negativ ist. Es können alternativ auch die Phase und die Amplitude kombiniert verwendet werden, so dass die Phase insbesondere die Information über das Vorzeichen der Abweichung liefert.

Vorteilhafterweise wird aus der Frequenz des Auslesesignals ein relativer Druck ermittelt, mit dem der kapazitive Drucksensor des Schwingkreises beaufschlagt ist. Es ist erfindungsgemäß nicht erforderlich, dass ein absoluter Druckwert bestimmt wird, vielmehr ist es ausreichend, einen relativen Druckwert zu ermitteln, der auf einen Referenzwert bezogen ist. Es können erfindungsgemäß also insbesondere Druckdifferenzen erfindungsgemäß ermittelt werden.

Vorteilhafterweise wird das erfindungsgemäße Verfahren mit einem wie vorstehend beschrieben erfindungsgemäßen Transpondersystem ausgeführt.

Im Folgenden soll die Erfindung anhand einiger Figuren beispielhaft erläutert werden. Gleiche oder entsprechende Merkmale sind mit gleichen Bezugszeichen gekennzeichnet. Die in den Beispielen beschriebenen Merkmale können auch unabhängig vom konkreten Beispiel realisiert sein und unter den Beispielen kombiniert werden.

Es zeigt:
- Figur 1: ein schematisches Blockschaltbild eines beispielhaften erfindungsgemäßen Transpondersystems,
- Figur 2: eine beispielhafte Implementierung eines erfindungsgemäßen Transpondersystems als Blockschaltbild,
- Figur 3: eine beispielhafte Implementierung einer Erzeugung eines Auslesesignals und einer Auswertung des Rückmeldesignals, wie sie in Figur 2 zum Einsatz kommen, in einem Regelkreis,
- Figur 4: eine beispielhafte Implementierung des in Figur 3 gezeigten Regelkreises,
- Figur 5: eine Darstellung von Phase und Amplitude, wie sie von einem Lock-in-Verstärker wie in Figur 3 und 4 gezeigt, ausgegeben werden können, sowie eines Kontrollsignals, abgeleitet aus der Phase und ihrer Ableitung.

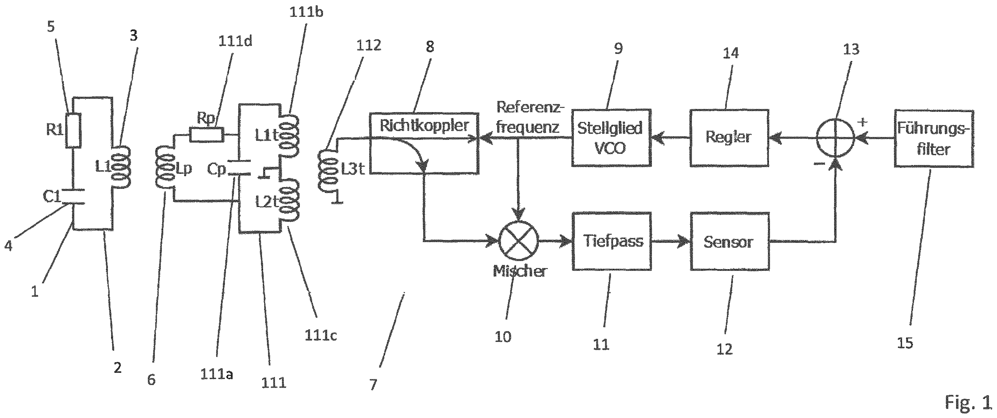

Figur 1 zeigt ein Beispiel eines erfindungsgemäßen Transpondersystems als schematisches Blockschaltbild. Das Transpondersystem weist einen passiven Transponder 1 auf, der einen Schwingkreis 2 mit veränderlicher Resonanzfrequenz enthält. Im gezeigten Beispiel weist der Schwingkreis 2 eine Induktivität 3 und eine Kapazität 4 auf. Für den Schwingkreis 2 ist außerdem ein Widerstand 5 eingezeichnet.

Das in Figur 1 gezeigte Transpondersystem weist außerdem eine Auslesevorrichtung 7 auf, die eine Ausleseinduktivität 6 enthält. Die Ausleseinduktivität ist hier an einen optionalen Balun-Übertrager 111 angeschlossen, der das nicht massenbezogene Signal der Auslesespule 6 (ein symmetrisches Signal) in ein massenbezogenes Signal umwandeln kann. Das vom Balun-Übertrager 111 erzeugte Signal wird hier dann an eine Sekundärspule 112 übertragen, die ein Signal erzeugt, das in einen massebezogenen Eingang eines Richtkopplers 8 eingespeist wird. Die Auslesespule weist im gezeigten Beispiel eine parasitäre Kapazität 111a auf. Zwischen der Auslesespule 6 und der Parallelschaltung aus der parasitären Kapazität 111a ist ein Widerstand 111d eingezeichnet. Er stellt den parasitären Widerstand der Auslesepule dar. Die Induktivitäten 111b und 111c sind zur Auslesespule 6 parallel geschaltet.

Eine Signalquelle 9 zur Erzeugung eines Wechselspannungssignals mit einer vorgegebenen Frequenz ist an einem Ausgang des Richtkopplers 8 angeschlossen. An einem zweiten Ausgang des Richtkopplers ist ein Mischer 10 angeordnet, der ein von der Ausleseinduktivität 6 oder dem Balun-Übertrager 111 in den Richtkoppler 8 einlaufendes Signal, das durch den zweiten Ausgang austritt, mit dem von der Signalquelle 9 erzeugten Wechselspannungssignal mischt. Das vom Mischer 10 ausgegebene Signal wird einem Tiefpassfilter 11 zugeführt, durch den beispielsweise Rausch- und Störanteile herausgefiltert werden. Das vom Tiefpassfilter ausgegebene Signal kann dann einem Sensor 12 zugeführt werden, mit dem Amplitude und/oder Phase des von der Auslesespule 6 empfangenen Signals aus dem vom Tiefpassfilter 11 ausgegebene Signal bestimmbar sind. Der Sensor 12 kann optional auch ein vom Tiefpassfilter 11 ausgegebenes komplexes Signal in ein reelles Signal umrechnen, das dann zur Regelung verwendet wird. Das vom Sensor 12 ausgegebene Signal kann dann in einem Komparator 13 mit einem von einem Führungsfilter 15 erzeugten Signal verglichen werden. Ist die Frequenzänderung der Schwingkreise 2a, 2b klein im Vergleich zur Bandbreite der Resonanz der Schwingkreise 2a, 2b, so eignet sich das Phasensignal vorteilhaft als Eingangsgröße für den Komparator 13. Das vom Komparator 13 ausgegebene Signal kann dann einem Regler 14 zugeführt werden, der ein Frequenzanpassungssignal an die Signalquelle 9 ausgibt, so dass die Frequenz des von der Signalquelle 9 erzeugten Signals basierend auf dem vom Regler 14 ausgegebenen Frequenzanpassungssignal angepasst werden kann. Auf diese Weise erlaubt der in Figur 1 gezeigte Regelkreis eine Anpassung der Frequenz des durch die Signalquelle 9 erzeugten Anregungssignals an eine veränderte Resonanzfrequenz des Schwingkreises 2. Im gezeigten Beispiel ist die Signalquelle 9 ein Spannungsgesteuerter Oszillator, der ein Signal mit einer Frequenz erzeugt, die von der angelegten Spannung, hier durch den Regler 14 erzeugt, abhängt.

Sofern der passive Transponder 1 nur einen Schwingkreis 2 aufweist, kann die Signalquelle 9 zum Auslesen der Schwingkreise ein einzelnes Sinussignal mit einer bestimmten Frequenz erzeugen. Sofern der passive Transponder 1 optional mehrere Schwingkreise 2 aufweist, können diese vorteilhafterweise voneinander verschiedene Frequenzen haben. In diesem Fall kann die Signalquelle 9 vorteilhafterweise ein Signal erzeugen, in dem eine der Zahl der unterschiedlichen Schwingkreise entsprechende Zahl an Sinusfunktionen mit den unterschiedlichen Frequenzen der mehreren Schwingkreise überlagert sind. Die unterschiedlichen Frequenzen können mit der Auslesespule 6 ausgelesen werden. Vorzugsweise wird das vom Richtkoppler 8 zum Mischer 10 ausgegebene Signal in diesem Fall mit den Sinussignalen mit den unterschiedlichen Resonanzfrequenzen getrennt gemischt. Es können hierzu vorteilhaft eine entsprechende Anzahl an getrennten Mischern 10 vorgesehen sein. Die auf diese Weise erzeugten getrennten Signale können dann jeweils einem Tiefpassfilter 11 und jeweils einem Sensor 12 zugeführt werden, der für alle Anteile jeweils die Amplitude und/oder Phase ermittelt. Es können dann für alle Frequenzanteile getrennte Rückkopplungen an die Signalquelle 9 erfolgen. In dem Fall, dass der passive Transponder 1 mehrere Schwingkreise 2 mit unterschiedlichen Resonanzfrequenzen aufweist, wird also vorzugsweise für jede der Resonanzfrequenzen ein eigener Mischer 10, ein eigener Tiefpassfilter 11, ein eigener Sensor 12, ein eigener Komparator 13, ein eigener Regler 14 und ein eigener Führungsfilter 15 und ein eigenes Stellglied 9 vorgesehen. Zusätzlich kann ein Addierer vorgesehen werden, der die von den Stellgliedern erzeugten Signale überlagert und gemeinsam an den Richtkoppler 8 ausgibt. Vorteilhaft ist insbesondere eine Ausgestaltung mit zwei Schwingkreisen 2, da diese eine Messung von Druckwellengeschwindigkeiten erlaubt.

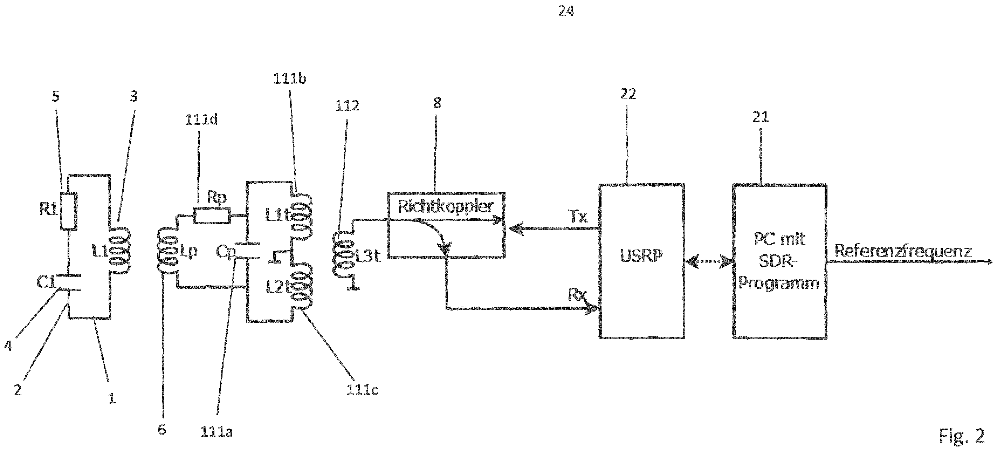

Figur 2 zeigt beispielhaft ein Blockschaltbild einer Implementierung des Regelkreises in Figur 1 zur Auslesung eines Transponders 1 mit einem Schwingkreis 2. Der Schwingkreis 2, der Balun-Übertrager 111, die Auslesespule 6 und der Richtkoppler 8 sind wie in Figur 1 gezeigt ausgestaltet. Auf die Beschreibung zur Figur 1 soll hier daher verwiesen werden. Der Mischer 10, der Tiefpassfilter 11, der Sensor 12, der Komparator 13, der Führungsfilter 15, der Regler 14 und das Stellglied 9 sind in diesem Beispiel in einem Rechner 21 implementiert, der mit dem Richtkoppler 8 über ein Universal Software Radio Peripheral (USRP) 22 verbunden ist. Die genannten Elemente können jedoch auch als einzelne digitale Bausteine oder als Analogschaltungen implementiert sein, was zu dem in Figur 1 gezeigte Blockschaltbild führen würde. Das USRP 22 überträgt ein Referenzsignal Tx an den einen Ausgang des Richtkopplers 8 und empfängt ein Rückmeldesignal Rx vom anderen Ausgang des Richtkopplers 8.

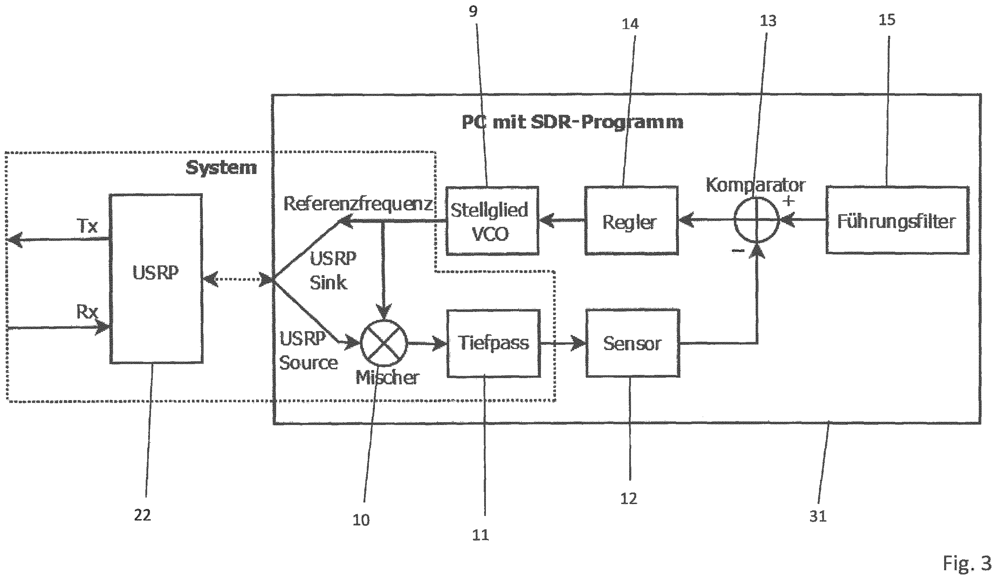

Figur 3 zeigt schematisch eine Implementierung einer Schaltung 31 zur Auswertung des von dem USRP 22 ausgegebenen Signals. Die Schaltung 31 kann dabei zum Beispiel mittels einer USB-Verbindung mit dem USRP 22 verbunden sein. Das Rückmeldesignal Rx wird dem Mischer 10 über eine USRP Quelle (USRP Source) zugeführt. Das Stellglied 9 führt dem USRP 22 das Auslesesignal Tx über eine USRP Senke (USRP Sink) zu. Der Mischer 10, der Tiefpass 11, der Sensor 12, der Komparator 13, der Führungsfilter 15, der Regler 14 und das Stellglied 9 sind wie in Figur 9 gezeigt verschaltet, so dass auf die Beschreibung dort verwiesen werden soll. Diese Elemente sind hier als Objekte einer Software realisiert, sie können aber auch als einzelne digitale Bauelemente oder als analog Bauelemente realisiert sein.

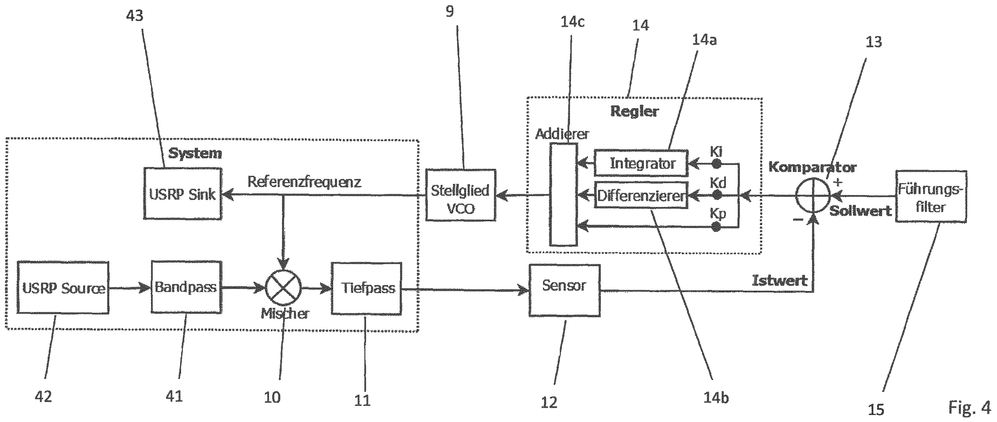

Figur 4 zeigt die in Figur 3 gezeigte Ausgestaltung der Erfindung mit einer detaillierten Darstellung des Reglers 14 und mit einem Bandpassfilter 41 hinter der USPR Quelle 42. Es wird hier das aus der USRP Quelle 42 ausgegebene Signal zunächst dem Bandpassfilter 41 zugeführt, der beispielsweise nur Frequenzen um eine bestimmte Mittelfrequenz passieren lässt und höhere und/oder tiefere Frequenzen als eine obere bzw. untere Grenzfrequenz entfernt. Das vom Bandpassfilter 41 ausgegebene Signal wird dann dem Mischer 10 zugeführt.

Im in Figur 4 gezeigten Beispiel weist der Regler 14 einen Integrator 14a auf, der über das vom Komparator 13 ausgegebene Signal über die Zeit integriert. Der Regler 14 weist außerdem einen Differenzierer 14b auf, der eine Ableitung des vom Komparator ausgegebenen Signals nach der Zeit berechnet.

Das vom Komparator ausgegebene Signal wird im Regler 14 dem Integrator 14a und dem Differenzierer 14b zugeführt. In einem Addierer 14c wird das vom Komparator ausgegebene Signal mit dem von Integrator 14a und vom Differenzierer 14b ausgegebenen Signal addiert und als Ergebnis der Addition eine Spannung erhalten, mittels derer der spannungsgesteuerte Oszillator 9 gesteuert wird. Das vom Komparator 13 ausgegebene Signal kann optional vor dem Einleiten in den Integrator 14a, den Differenzierer 14b und in den Addierer 14c jeweils mit einem konstanten Wert multipliziert werden. In Figur 4 kann das in den Integrator 14a eingeleitete Signal mit einem Wert Ki multipliziert werden, dass in den Differenzierer eingeleitete Signal mit einem Wert Kd und das direkt dem Addierer 14c zugrührte Signal mit einem Wert Kp.

Die Konstanten Ki, Kd und Kp können an das zu regelnde System angepasst werden. Über sie kann der Regler so eingestellt werden, dass sich Regler und System im Gesamten stabil verhalten. Es sollte hierzu ein Optimum zwischen Regelgeschwindigkeit und Genauigkeit gefunden werden. Beispielhaft wurde zu diesem Zweck die Sprungantwort des Systems in einer open loop Konfiguration ermittelt. Dazu wurde das Gesamtsystem, das in Fig. 3 dargestellt ist, am Eingang des Stellglieds 9 mit einer Sprungfunktion beaufschlagt. Der Regler 14 wurde entfernt und der Kreis somit an dieser Stelle unterbrochen. Die Sprungantwort ist dann am Ausgang des Komparators 13 zu messen. Anhand der Sprungantwort kann dann beispielsweise mit der Methode nach Ziegler und Nichols (Ziegler, J. G.; Nichols, N. B.: Optimum settings for automatic controllers, Trans. ASME, 64 (1942), pp. 759-768) eine geeignete Parameterkombination für Ki, Kd und Kp gefunden werden.

Da der Regler 14 dem Signal folgt und der Signalquelle 9 bzw. dem Stellglied 9 stets die aktuell einzustellende Frequenz in Form eines Spannungswertes liefert, kann diese auch dort, also zwischen dem Regler 14 und dem Stellglied 9 in Fig. 4) abgegriffen werden.

Figur 5 zeigt beispielhaft einen Verlauf einer Phase 51, einer Amplitude 52 und eines Kontrollsignals 53, wie sie vom Sensor 12 in den Figuren 1 bis 4 erzeugt werden können. Der Mittelpunkt des Diagramms ist dabei in Frequenzrichtung (horizontale Richtung) die eingespeiste Anregungsfrequenz und in vertikaler Richtung ein Nullpunkt eines normierten Phasen, Amplituden bzw. Kontrollsignals. Das Phasensignal weist um den Nullpunkt in der Mitte des Diagramms einen linearen Bereich auf, in dem aus der Phase direkt auf die Abweichung des Rückmeldesignals vom Auslesesignal geschlossen werden kann. Die Amplitude ist im die Frequenz des Auslesesignals symmetrisch, so dass für eine Regelung zusätzliche Informationen herangezogen werden sollten, die Auskunft darüber geben, in welcher Richtung das Rückmeldesignal vom Auslesesignal abweicht. Dies kann zum Beispiel die Phase oder aber auch eine Ableitung der Amplitude nach der Frequenz sein, deren Vorzeichen vom Vorzeichen der Abweichung abhängt. Während das Phasensignal insbesondere für kleine Frequenzabweichungen geeignet ist, kann die Abweichung im Kontrollsignal über einen weiten Bereich von Frequenzabweichungen ermittelt werden.

Das Kontrollsignal kann zum Beispiel wie im folgenden beschrieben erzeugt werden. Die Verwendung des Kontrollsignals ist vorteilhaft, weil es über den ganzen Bereich von Frequenzabweichungen eindeutig definiert, ob die tatsächliche Resonanzfrequenz unterhalb oder oberhalb der Anregungsfrequenz liegt. Insbesondere bei Resonanzfrequenzänderungen von mehr als f(min(z_phase)) bis f(max(z_phase)) ist vorteilhaft.

Da es sich um eine Echtzeitsignalverarbeitung mit einem sich mit der Pulsfrequenz (z.B. ca. 70 Hz) wiederholenden Signal handelt, können zum Beispiel in einem ersten Schritt über eine oder mehrere Perioden die Parameter (z.B. Phasenminimum z_phase_min und Phasenmaximum z_phase_max) ermittelt werden, die für die Erzeugung der Regelgröße control_out verwendet werden können. Im zweiten Schritt kann dann die Echtzeitverarbeitung mit den zuvor gewonnenen Parametern erfolgen. Auch eine adaptive Gewinnung der Parameter in Echtzeit wäre möglich.

In einer beispielhaften Ausführungsform, bei der die Regelgröße aus der Phase bestimmt wird, kann zum Beispiel folgender Ablauf verwendet werden, der hier als Pseudo-Code wiedergegeben ist:

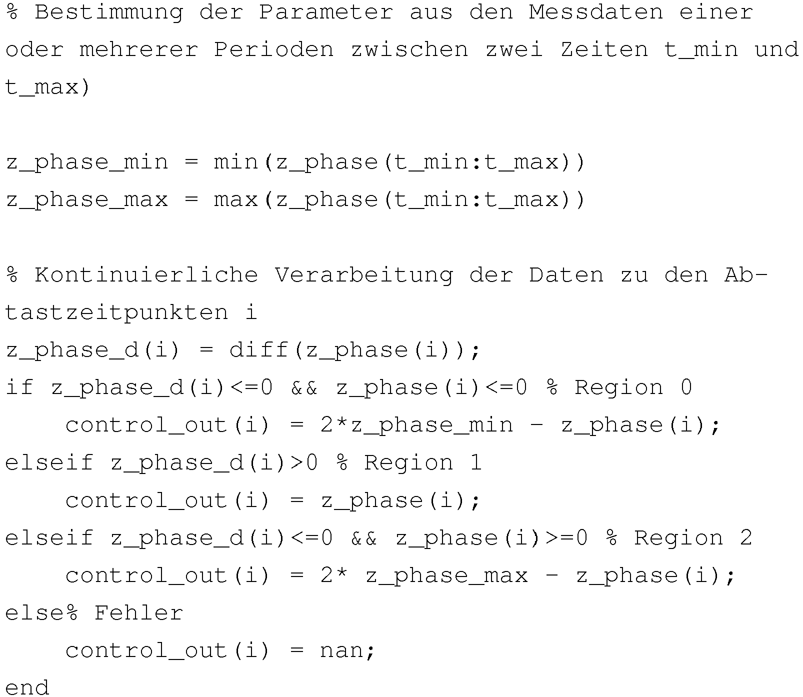

Dabei bezeichnet z_phase die Phase, z_phase_d die Ableitung der Phase und control_out den Wert des Kontrollsignals.

Die Erfindung kann beispielsweise den zeitlich veränderlichen Druck in Blutgefäßen messen. Hierzu kann der Schwingkreis 2 mit einem kapazitiven Drucksensor 4 und einer Spule 3, die gleichzeitig für die Kopplung mit der Auslesespule 6 verwendet wird, in das Blutgefäß eingebracht werden, so dass sie dort dem zeitlich veränderten Druck ausgesetzt ist. Über eine extrakorporal induktiv angekoppelte Auslesespule 6 kann dann eine Anregungsfrequenz, die die Resonanzfrequenz des Sensors bei einem bestimmten Druck ist, eingespeist werden und das Feedback beobachtet werden. Mittels der Erfindung können vorteilhaft alle auftretenden Drücke über die Zeit gemessen werden, so dass eine kontinuierliche Messung des Druckes möglich ist. Hierdurch sind auch Größen mit größerer Genauigkeit bestimmbar, die aus einem oder mehreren Drücken bestimmbar sind, wie beispielsweise eine Pulswellengeschwindigkeit. Da ein größerer Teil des Druckverlaufs gemessen werden kann, können mehr Datenpunkte ermittelt werden, so dass die Pulswellengeschwindigkeit mit höherer Genauigkeit ermittelbar ist, als wenn nur ein Triggerdruck verwendet wird.

Die Erfindung kann als Analogschaltung oder mittels digitaler Signalverarbeitung realisiert sein, beispielsweise als SDR mit Frequenzumsetzung oder als Analog-Digital-Konverter mit DA-Konverter ohne Frequenzumsetzung. Die Erfindung kann vorteilhaft bei allen Anwendungen eingesetzt werden, bei denen induktive Kopplung zwischen Sensoren, insbesondere implantierten Sensoren, und einer Ausleseeinheit verwendet wird. Es können auf diese Weise besonders vorteilhaft periodische oder nicht-periodische Signale erfasst werden.

## Patentansprüche

1. Transpondersystem zur Messung von Druck in Fluidleitungen, aufweisend
zumindest einen passiven Transponder (1),
wobei der passive Transponder (1) zumindest einen Schwingkreis (2) mit einer veränderlichen Resonanzfrequenz aufweist,
sowie zumindest eine Auslesevorrichtung (7),
wobei die Auslesevorrichtung (7) zumindest eine Ausleseinduktivität (6) aufweist,
wobei mit der Ausleseinduktivität (6) der zumindest eine Schwingkreis (2) mit einem Auslesesignal beaufschlagbar ist,
wobei die Auslesevorrichtung (7) eingerichtet ist, eine Frequenz des Auslesesignals an die veränderliche Resonanzfrequenz des Schwingkreises (2) anzupassen,
**dadurch gekennzeichnet, dass** zumindest ein induktiver Sensor (3) und zumindest eine Kapazität (4) zu dem zumindest einen Schwingkreis (2) verschaltet sind.

2. Transpondersystem nach dem vorhergehenden Anspruch, wobei die Auslesevorrichtung (7) eingerichtet ist, die Frequenz des Auslesesignals der veränderlichen Resonanzfrequenz des Schwingkreises (2) stetig nachzuführen.

3. Transpondersystem nach einem der vorhergehenden Ansprüche, wobei die Auslesevorrichtung
eine Anregungseinheit zur Erzeugung des Auslesesignals und
eine Rückmeldeeinheit zur Erfassung eines vom passiven Transponder ausgehenden Rückmeldesignals aufweist,
wobei die Rückmeldeeinheit eingerichtet ist, ein Frequenzanpassungssignal an die Anregungseinheit zu übergeben, das basierend auf dem Rückmeldesignal ermittelt wird,
und wobei die Anregungseinheit eingerichtet ist, die Frequenz des Auslesesignals basierend auf dem Frequenzanpassungssignals anzupassen.

4. Transpondersystem nach dem vorhergehenden Anspruch,
wobei die Anregungseinheit eine Signalquelle (9) aufweist, mit der das Auslesesignal erzeugbar ist,
wobei die Ausleseeinheit außerdem einen Richtkoppler (8) aufweist,
an dessen Ausgang die Signalquelle (9) elektrisch gekoppelt ist und an dessen Eingang die Ausleseinduktivität (6) elektrisch gekoppelt ist.

5. Transpondersystem nach einem der beiden vorhergehenden Ansprüche,
wobei die Rückmeldeeinheit einen Mischer (10) aufweist, wobei mit dem Mischer ein durch die Ausleseinduktivität (6) von dem passiven Transponder empfangenes Signal mit dem Auslesesignal mischbar oder multiplizierbar ist, wobei vorzugsweise die Rückmeldeeinheit außerdem einen Tiefpassfilter (11) aufweist, mit dem ein vom Mischer (10) ausgegebenes Signal filterbar ist.

6. Transpondersystem nach einem der Ansprüche 3 bis 5,
wobei die Rückmeldeeinheit zusammen mit der Anregungseinheit einen Regelkreis bildet.

7. Verfahren zum Auslesen eines passiven Transponders,
wobei der passive Transponder zumindest einen Schwingkreis mit einer veränderlichen Resonanzfrequenz aufweist,
wobei der zumindest eine Schwingkreis mit einem Auslesesignal beaufschlagt wird,
wobei eine Frequenz des Auslesesignals an die veränderliche Resonanzfrequenz des Schwingkreises angepasst wird, wobei das Verfahren mit einem Transpondersystem nach einem der Ansprüche 1 bis 6 ausgeführt wird.

8. Verfahren nach dem vorhergehenden Anspruch,
wobei die Frequenz des Auslesesignals der veränderlichen Resonanzfrequenz des Schwingkreises stetig nachgeführt wird.

9. Verfahren nach einem der beiden vorhergehenden Ansprüche,
wobei die Frequenz des Auslesesignals mittels eines Regelkreises so nachgeführt wird, dass die Frequenz des Auslesesignals die jeweils gegenwärtige Resonanzfrequenz des Schwingkreises ist.

10. Verfahren nach einem der drei vorhergehenden Ansprüche,
wobei ein vom passiven Transponder ausgehendes Rückmeldesignal empfangen wird, und das Rückmeldesignal einem Eingang eines Lock-in-Verstärkers zugeführt wird, wobei ein Referenzsignal des Lock-in-Verstärkers das Auslesesignal ist.

11. Verfahren nach einem der Ansprüche 7 bis 10,
wobei aus der Frequenz des Auslesesignals ein relativer Druck ermittelt wird, mit dem ein als Kapazität des Schwingkreises wirkender Drucksensor beaufschlagt ist.

## Claims

1. A transponder system for measuring pressure in fluid lines, said system comprising
at least one passive transponder (1), wherein the passive transponder (1) comprises at least one resonant circuit (2) with a variable resonance frequency, and at least one readout device (7), wherein the readout device (7) comprises at least one readout inductor (6),
wherein a readout signal may be applied to the at least one resonant circuit (2) using the readout inductor (6),
wherein the readout device (7) is configured to adapt a frequency of the readout signal to the variable resonance frequency of the resonant circuit (2),
**characterized in that** at least one inductive sensor (3) and at least one capacitor (4) are connected to form the at least one resonant circuit (2).

2. The transponder system according to the preceding claim, wherein the readout device (7) is configured to continuously track the frequency of the readout signal of the variable resonance frequency of the resonant circuit (2).

3. The transponder system according to any one of the preceding claims, wherein the readout device comprises
an excitation unit for generating the readout signal and
a feedback unit for detecting a feedback signal originating from the passive transponder,
wherein the feedback unit is configured to transfer a frequency matching signal to the excitation unit, which frequency matching signal is determined on the basis of the feedback signal,
and wherein the excitation unit is configured to match the frequency of the readout signal on the basis of the frequency matching signal.

4. The transponder system according to the preceding claim,
wherein the excitation unit comprises a signal source (9), by means of which the readout signal may be generated,
wherein the readout unit additionally comprises a directional coupler (8), to the output of which the signal source (9) is electrically coupled and to the input of which the readout inductor (6) is electrically coupled.

5. The transponder system according to any one of the two preceding claims,
wherein the feedback unit comprises a mixer (10), wherein a signal received from the passive transponder by the readout inductor (6) may be mixed or may be multiplied with the readout signal by means of the mixer, wherein preferably the feedback unit additionally comprises a low-pass filter (11), by means of which a signal output by the mixer (10) may be filtered.

6. The transponder system according to any one of claims 3 to 5,
wherein the feedback unit together with the excitation unit forms a control loop.

7. A method for reading out a passive transponder,
wherein the passive transponder has at least one resonant circuit with a variable resonance frequency,
wherein a readout signal is applied to the at least one resonant circuit,
wherein a frequency of the readout signal is matched to the variable resonance frequency of the resonant circuit, wherein the method is carried out with a transponder system according to any one of claims 1 to 6.

8. The method according to the preceding claim,
wherein the frequency of the readout signal of the variable resonance frequency of the resonant circuit is continuously tracked.

9. The method according to any one of the two preceding claims,
wherein the frequency of the readout signal is tracked by means of a control loop such that the frequency of the readout signal is the respective current resonance frequency of the resonant circuit.

10. The method according to any one of the three preceding claims,
wherein a feedback signal originating from the passive transponder is received, and the feedback signal is fed to an input of a lock-in amplifier, wherein a reference signal of the lock-in amplifier is the readout signal.

11. The method according to any one of claims 7 to 10,
wherein a relative pressure is determined from the frequency of the readout signal and is applied to a pressure sensor acting as capacitor of the resonant circuit.

## Revendications

1. Système de transpondeur permettant de mesurer la pression dans des conduites de fluide, comprenant
au moins un transpondeur passif (1),
dans lequel le transpondeur passif (1) présente au moins un circuit oscillant (2) avec une fréquence de résonance variable,
et au moins un dispositif de lecture (7),
dans lequel le dispositif de lecture (7) présente au moins une inductance de lecture (6),
dans lequel le au moins un circuit oscillant (2) peut être soumis à un signal de lecture par l'inductance de lecture (6),
le dispositif de lecture (7) étant conçu pour adapter une fréquence du signal de lecture à la fréquence de résonance variable du circuit oscillant (2),
**caractérisé en ce qu'**au moins un capteur inductif (3) et au moins un condensateur (4) sont connectés à au moins un circuit oscillant (2).

2. Système de transpondeur selon la revendication précédente, dans lequel le dispositif de lecture (7) est conçu pour actualiser en permanence la fréquence du signal de lecture de la fréquence de résonance variable du circuit oscillant (2).

3. Système de transpondeur selon l'une quelconque des revendications précédentes,
dans lequel le dispositif de lecture comprend
une unité d'excitation permettant de générer le signal de lecture et
une unité de rétroaction permettant de détecter un signal de rétroaction émanant du transpondeur passif,
l'unité de rétroaction étant conçue pour transmettre à l'unité d'excitation un signal d'adaptation de fréquence qui est déterminé en se basant sur le signal de rétroaction,
et l'unité d'excitation étant conçue pour adapter la fréquence du signal de lecture en se basant sur le signal d'adaptation de fréquence.

4. Système de transpondeur selon la revendication précédente,
dans lequel l'unité d'excitation comprend une source de signal (9) avec laquelle le signal de lecture peut être généré,
dans lequel l'unité de lecture comprend en outre un coupleur directionnel (8) à la sortie duquel la source de signal (9) est couplée électriquement et à l'entrée duquel l'inductance de lecture (6) est couplée électriquement.

5. Système de transpondeur selon l'une quelconque des deux revendications précédentes,
lequel comprend une unité de rétroaction dotée d'un mélangeur (10), ce mélangeur permettant de mélanger ou de multiplier un signal reçu par l'inductance de lecture (6) du transpondeur passif avec le signal de lecture, de préférence, l'unité de rétroaction comprend également un filtre passe-bas (11) permettant de filtrer un signal émis par le mélangeur (10).

6. Système de transpondeur selon l'une quelconque des revendications 3 à 5,
dans lequel l'unité de rétroaction forme avec l'unité d'excitation une boucle de régulation.

7. Procédé de lecture d'un transpondeur passif,
dans lequel le transpondeur passif présente au moins un circuit oscillant avec une fréquence de résonance variable,
dans lequel le au moins un circuit oscillant est soumis à un signal de lecture,
dans lequel une fréquence du signal de lecture est adaptée à la fréquence de résonance variable du circuit oscillant, le procédé étant mis en œuvre grâce à un système de transpondeur selon l'une quelconque des revendications 1 à 6.

8. Procédé selon la revendication précédente,
dans lequel la fréquence du signal de lecture de la fréquence de résonance variable du circuit oscillant est actualisée en permanence.

9. Procédé selon l'une quelconque des revendications précédentes,
dans lequel la fréquence du signal de lecture est actualisée au moyen d'un circuit de régulation, de sorte que la fréquence du signal de lecture est la fréquence de résonance respectivement actuelle du circuit oscillant.

10. Procédé selon l'une quelconque des revendications précédentes,
dans lequel un signal de rétroaction émanant du transpondeur passif est reçu, et le signal de rétroaction est acheminé vers une entrée d'un amplificateur à verrouillage, le signal de référence de l'amplificateur à verrouillage étant le signal de lecture.

11. Procédé selon l'une quelconque des revendications 7 à 10,
dans lequel une pression relative à laquelle est soumis un capteur de pression agissant comme un condensateur du circuit oscillant est déterminée à partir de la fréquence du signal de lecture.
